# EUROPEAN PATENT SPECIFICATION

(11) **EP 1 990 017 B1**
(45) Date of publication and mention of the grant of the patent: **04.11.2009**
(21) Application number: 07009158.2
(22) Date of filing: 07.05.2007
(51) Int. Cl.: A61B 17/80

(54) **Sliding plate with reinforced slot**
Gleitplatte mit verstärktem Schlitz
Plaque coulissante à rainure renforcée

(43) Date of publication of application: 12.11.2008
(73) Proprietor: Stryker Trauma GmbH, 24232 Schönkirchen/Kiel (DE)
(72) Inventor: Van Helfteren, Alwin, 79102 Freiburg (DE)
(74) Representative: Kopf, Korbinian Paul

(56) References cited:
- EP-A- 1 468 656
- DE-A1- 10 125 092
- DE-U1-202005 019 277
- US-A- 6 096 040
- US-A1- 2002 128 653
- US-A1- 2006 200 145

## Description

### BACKGROUND OF THE INVENTION

The present invention relates to a bone plate, in particular, a bone plate for maintaining relative position of two pieces of fractured jaw bone.

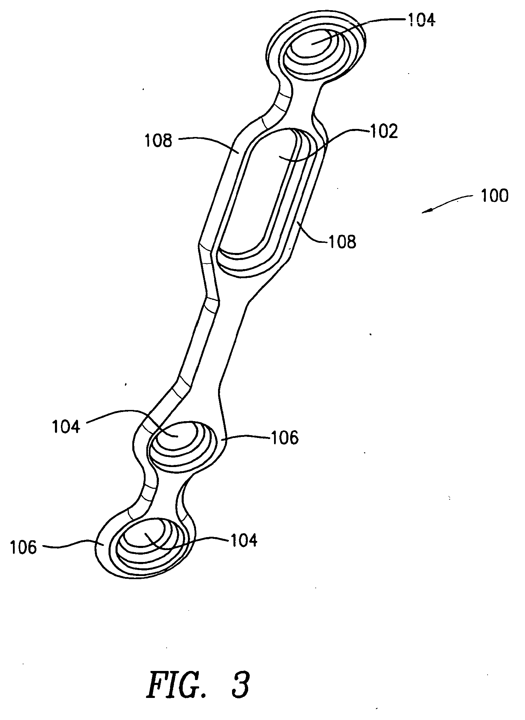

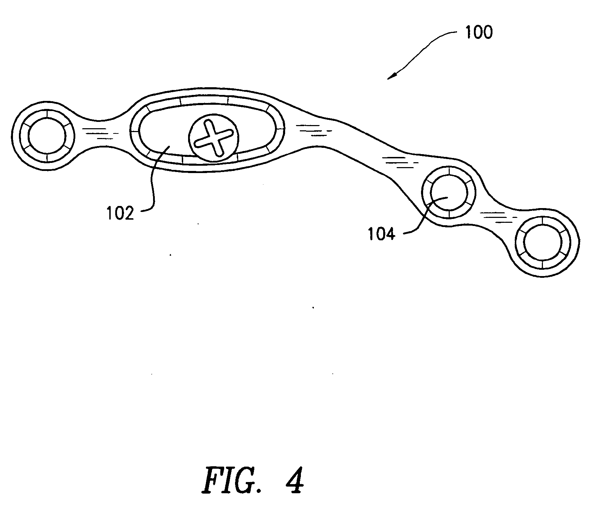

Bone plates are widely used to maintain fractured bones in a fixed relative position. Figure 3 shows a conventional bone plate 100. Conventional bone plate 100 has a slot 102 and holes 104. Holes 104 are surrounded by walls 106 and slot 102 is surrounded by walls 108. The diameter of holes 104 and the width of slot 102 are same. Since the maximum width of the bone plate measured at holes 104 and slot 102 are equal, the thickness of walls 106 and 108 are same. Often the nature and location of the fractures, and the bone contour at the fracture site, may require bending of plate 100 for implanting it as desired by the surgeon. Plate 100 may be bent side to side in the plane of plate 100 and additionally may also be bent transverse to the plane of plate 100. In conventional plates 100, when the plate is bent, as shown in Figure 4, slot 102 can deform. Often when slot 102 deforms, the opening of slot 102 is, at portions, larger than at other portions and often larger than the head of the fastener used in slot 102.

DE 101 25 092 A1 discloses a Y-shaped bone plate, wherein several holes are formed in each shank of the 'Y'. Further, there is provided a slot in the central portion of the plate, in which the two shanks are connected to each other as well as to the base of the 'Y', such that the wall thickness of the slot is greater in the direction to the shanks.

DE 20 2005 019 277 U1 discloses a bone plate having two slot and several holes, wherein the slots are perpendicularly orientated to each other. The walls surrounding the holes and the slots are similar, such that the above described disadvantages can be found also in said plate.

Further, US 2002/0128653 A1 describes an acetabulum bone plate including a central support portion which is pre-shaped to cover the posterior wall of the acetabulum, and a pair of tabs which extend therefrom and attach to the pelvis. Said bone plate is not intended to be bended and to be used in other fracture situations.

Therefore, there is a need for an improved bone plate wherein the slot would not get deformed when it is necessary to bend the plate.

### SUMMARY OF THE INVENTION

The present invention provides a bone plate that may be used to maintain relative position of two pieces of fractured bone, for example, a fractured jaw bone. The bone plate of present invention has a slot and holes. The holes are surrounded by walls and the slot is also surrounded by walls. The thickness of walls surrounding the slot at their maximum is greater than the thickness of walls surrounding the holes.

In use, the bone plate is first attached to a first fragment of fractured bone. Next, bone fragments are aligned and the other end of the bone plate is attached to second fragment. Since the attachment to the second fragment is via a screw inserted in the slot, the first fragment and the second fragment can be moved relative to each other. Once the first fragment and the second fragment are brought in relative positions desired by the surgeon, additional screw are inserted into second fragment and first fragment. Often the nature of the fracture may require bending of the plate for implanting it as desired by the surgeon. The plate of present invention has an elongated neck portion where the plate may be bent. Because walls of the slot are designed to be strong, the slot is not deformed when the plate is bent.

### BRIEF DESCRIPTION OF THE DRAWINGS

Figure 1 shows a plan view of an embodiment of a bone plate of present invention.
Figure 2 shows a pictorial view of a bone plate implanted on a jaw bone.
Figure 3 shows a conventional bone plate.
Figure 4 shows a conventional plate that has been bent.

### DETAILED DESCRIPTION

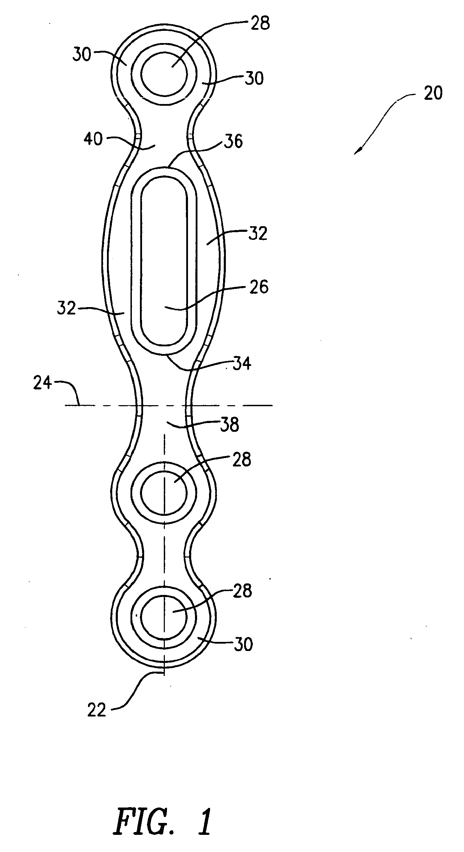

Figure 1 shows a bone plate 20. Bone plate 20 may be used to maintain relative position of two pieces of fractured bone, for example, a fractured jaw bone. Bone plate 20 is an elongate part made from bio-compatible material. Bone plate 20 has a longitudinal axis 22 and a transverse axis 24. Bone plate 20 has a slot 26 and holes 28. Holes 28 may be located on one side or both side of slot 26. Bone plate 20 may have one or more holes 28. The Holes 28 are surrounded by walls 30 and slot 26 is surrounded by walls 32. The outer contour of plate 20 around holes 28 may be circular and the outer contour of plate 20 around slot 28 may be arcuate. The outer contours of the plate may take other shapes. In the embodiment of Figure 1 having the outer contour around holes 28 circular and the outer contour around slot 26 arcuate, the thickness of the walls 30 are measured radially from the centre of the corresponding holes 28 and the thickness of the walls 32 are measured parallel to transverse axis 24. The thicknesses of walls 30 are constant whereas the thicknesses of the walls 32 vary at different points along the length of slot 26 though in a preferred embodiment it is not necessary that the walls 32 be different in thickness at every point. In a preferred embodiment, the max thickness is at the central region of the slot 26. The thickness of walls 32 at their maximum is greater than the thickness of walls 30. In another embodiment, wall 32 may have a constant thickness that is greater than walls 30. Slot 26 has a first end 34 and a second end 36. A hole 28 that is located near the first end 34 is connected to the first end 34 via a neck 38. A hole 28 that is located near the second end 36 is connected to the second end 34 via a neck 40. The width of bone plate 20 is measured parallel to the transverse axis 24 and is narrower in the neck area.

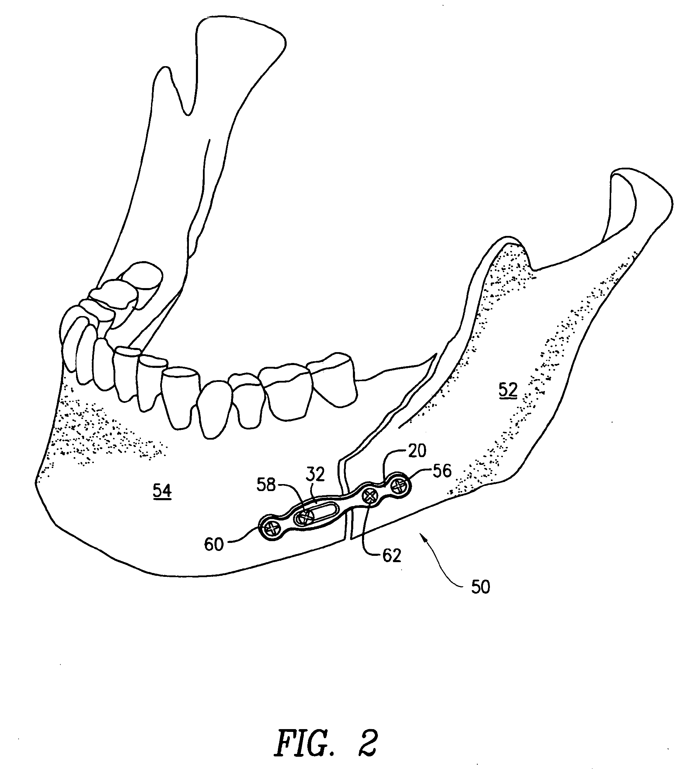

Figure 2 shows a bone plate 20 attached to first fragment 52 and second fragment 54 of a fractured jaw bone 50. In use, bone plate 20 may be first attached to first fragment 52 of fractured bone 50 by placing one hole 28 of bone plate 20 on the first fragment 52 and inserting a screw 56 through hole 28 and into bone 50. Next, bone fragments 52 and 54 may be aligned and the other end of bone plate 20 may be attached to second fragment 54 by inserting screw 58 through slot 26 and in second fragment 54 of bone 50. Since screw 58 is inserted in a slot, it gives the surgeon flexibility to reduce the fracture, i.e., align first fragment 52 and the second fragment 54 after screw 58 is inserted. Once first fragment 52 and the second fragment 54 are brought in relative positions desired by the surgeon, additional screw 60 and thereafter screw 62 are inserted via respective holes 28 into second fragment 54 and first fragment 52 respectively. Often the nature and location of the fracture, and the contour of the bone site, may require bending of plate 20 for implanting it as desired by the surgeon. Plate 20 has an elongated neck portion 64 where the plate may be bent. Plate 20 may be bent in the plane of plate 20 or transverse to the plane of plate 20. Plate 20 may also be bent in any other manner deemed necessary by the surgeon. The walls 32 of slot 26 are designed to withstand the bending forces which might be translated to this region as the elongated neck portion 64 is bent. Thus, slot 26 is not deformed when plate 20 is bent intraoperatively to be contoured to the bone at or adjacent a fracture site.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A bone plate (20) for maintaining relative position of two pieces of fractured bone, the bone plate comprising:
an elongate plate having a longitudinal axis (22) and a transverse axis (24);
a slot (26) formed in the elongate plate, a length of the slot being parallel to the longitudinal axis of the plate, a width of the slot being parallel to the transverse axis, the slot having a first end (34) and a second end (36) in the direction of the longitudinal axis, and side walls (32) in the direction of the transverse axis, wherein each side wall (32) comprises an inner side, the inner side being straight; and an outer side, the outer side being generally in the shape of an arc, wherein the maximum thickness of each side wall (32) is between the inner side and the outer side,
a first annular hole (28) formed in the plate, the first annular hole being on the first end side (34) of the slot (26) and being surrounded by a first hole wall (30); and
a first neck portion (38) connecting the first annular hole (28) to the first end (34) of the slot (26), the neck portion being bendable in the plane of the plate and transverse to the plane of the plate,
wherein the maximum thickness of each side wall (32) is greater than a maximum thickness of the first hole wall (30) in the direction of the transverse axis.

2. The bone plate (20) of claim 1, wherein the bone plate is straight, the plate further comprises a second annular hole (28), the second hole being located on the second end side (36) of the slot, and a second neck portion (40) located between the slot and the second annular hole.

3. The bone plate (20) of claim 2, wherein the second hole (28) is surrounded by a second hole wall (30); and the maximum thickness of each side wall (32) is greater than a thickness of the second hole wall (30) in the direction of the transverse axis.

4. The bone plate (20) of claim 2, wherein the first neck portion (38) and second neck portion (40) have different lengths.

5. The bone plate (20) of claim 2, wherein the second neck portion (40) being shorter than the first neck portion (38).

6. The bone plate (20) of one of claims 1 to 5, wherein the each side wall (32) is thickest in a central region of the slot in the direction of the longitudinal axis.

7. The bone plate (20) of one of claims 1 to 6, further comprising:
a third hole (28) formed in the plate, the third hole being located on the first end side (34) of the slot or the second end side (36) of the slot.

8. The bone plate (20) of claim 1, wherein the plate is bendable at the first neck.

9. The bone plate (20) of claim 2, wherein the plate is bendable at the second neck.

10. The bone plate (20) of one of claims 1 to 2, wherein the plate is bendable in the plane of the plate and transverse to the plane of the plate.

## Patentansprüche

1. Knochenplatte (20) zum Aufrechterhalten der verhältnismäßigen Stellung zweier Stücke eines gebrochenen Knochens, wobei die Knochenplatte Folgendes umfasst:
eine längliche Platte, die eine Längsachse (22) und eine Querachse (24) hat,
einen Schlitz (26), der in der länglichen Platte geformt ist, wobei eine Länge des Schlitzes parallel zu der Längsachse der Platte ist, wobei eine Breite des Schlitzes parallel zu der Querachse ist, wobei der Schlitz ein erstes Ende (34) und ein zweites Ende (36) in der Richtung der Längsachse und Seitenwände (32) in der Richtung der Querachse hat, wobei jede Seitenwand (32) ein Innenseite, wobei die Innenseite gerade ist, und eine Außenseite, wobei die Außenseite im Allgemeinen die Form eines Bogens hat, umfasst, wobei sich die maximale Dicke jeder Seitenwand (32) zwischen der Innenseite und der Außenseite befindet,
ein erstes ringförmiges Loch (28), das in der Platte geformt ist, wobei sich das erste ringförmige Loch auf der Seite des ersten Endes (34) des Schlitzes (26) befindet und durch eine erste Lochwand (30) umschlossen wird, und
einen ersten Halsabschnitt (38), der das erste ringförmige Loch (28) mit dem ersten Ende (34) des Schlitzes (26) verbindet, wobei der Halsabschnitt in der Ebene der Platte und quer zu der Ebene der Platte gebogen werden kann,
wobei die maximale Dicke jeder Seitenwand (32) größer ist als eine maximale Dicke der ersten Lochwand (30) in der Richtung der Querachse.

2. Knochenplatte (20) nach Anspruch 1, wobei die Knochenplatte gerade ist, wobei die Platte ferner ein zweites ringförmiges Loch (28), wobei das zweite Loch auf der Seite des zweiten Endes (36) des Schlitzes angeordnet ist, und einen zweiten Halsabschnitt (40), der zwischen dem Schlitz und dem zweiten ringförmigen Loch angeordnet ist, umfasst.

3. Knochenplatte (20) nach Anspruch 2, wobei das zweite Loch (28) durch eine zweite Lochwand (30) umschlossen wird und die maximale Dicke jeder Seitenwand (32) größer ist als eine Dicke der zweiten Lochwand (30) in der Richtung der Querachse.

4. Knochenplatte (20) nach Anspruch 2, wobei der erste Halsabschnitt (38) und der zweite Halsabschnitt (40) unterschiedliche Längen haben.

5. Knochenplatte (20) nach Anspruch 2, wobei der zweite Halsabschnitt (40) kürzer ist als der erste Halsabschnitt (38).

6. Knochenplatte (20) nach einem der Ansprüche 1 bis 5, wobei jede Seitenwand (32) am dicksten in einem Mittelbereich des Schlitzes in der Richtung der Längsachse ist.

7. Knochenplatte (20) nach einem der Ansprüche 1 bis 6, die ferner Folgendes umfasst:
ein drittes Loch (28), das in der Platte geformt ist, wobei das dritte Loch auf der Seite des ersten Endes (34) des Schlitzes oder der Seite des zweiten Endes (36) des Schlitzes angeordnet ist.

8. Knochenplatte (20) nach Anspruch 1, wobei die Platte an dem ersten Hals gebogen werden kann.

9. Knochenplatte (20) nach Anspruch 2, wobei die Platte an dem zweiten Hals gebogen werden kann.

10. Knochenplatte (20) nach einem der Ansprüche 1 bis 2, wobei die Platte in der Ebene der Platte und quer zu der Ebene der Platte gebogen werden kann.

## Revendications

1. Plaque osseuse (20) destinée à maintenir la position relative de deux morceaux d'os fracturé, la plaque osseuse comportant :
une plaque allongée ayant un axe longitudinal (22) et un axe transversal (24),
une fente (26) formée dans la plaque allongée, une longueur de la fente étant parallèle à l'axe longitudinal de la plaque, une largeur de la fente étant parallèle à l'axe transversal, la fente ayant une première extrémité (34) et une seconde extrémité (36) dans la direction de l'axe longitudinal, et des parois latérales (32) dans la direction de l'axe transversal, dans laquelle chaque paroi latérale (32) comporte un côté intérieur, le côté intérieur étant rectiligne, et un côté extérieur, le côté extérieur ayant généralement la forme d'un arc, dans laquelle l'épaisseur maximale de chaque paroi latérale (32) se situe entre le côté intérieur et le côté extérieur,
un premier trou annulaire (28) formé dans la plaque, le premier trou annulaire étant du côté de la première extrémité (34) de la fente (26) et étant entouré d'une première paroi de trou (30), et
une première partie d'encolure (38) reliant le premier trou annulaire (28) à la première extrémité (34) de la fente (26), la partie d'encolure pouvant être pliée dans le plan de la plaque et transversale au plan de la plaque,
dans laquelle l'épaisseur maximale de chaque paroi latérale (32) est plus grande qu'une épaisseur maximale de la première paroi de trou (30) dans la direction de l'axe transversal.

2. Plaque osseuse (20) selon la revendication 1, dans laquelle la plaque osseuse est rectiligne, la plaque comportant en outre un deuxième trou annulaire (28), le deuxième trou étant situé du côté de la seconde extrémité (36) de la fente, et une seconde partie d'encolure (40) située entre la fente et le deuxième trou annulaire.

3. Plaque osseuse (20) selon la revendication 2, dans laquelle le deuxième trou (28) est entouré d'une seconde paroi de trou (30), et l'épaisseur maximale de chaque paroi latérale (32) est plus grande qu'une épaisseur de la seconde paroi de trou (30) dans la direction de l'axe transversal.

4. Plaque osseuse (20) selon la revendication 2, dans laquelle la première partie d'encolure (38) et la seconde partie d'encolure (40) ont des longueurs différentes.

5. Plaque osseuse (20) selon la revendication 2, dans laquelle la seconde partie d'encolure (40) est plus courte que la première partie d'encolure (38).

6. Plaque osseuse (20) selon l'une des revendications 1 à 5, dans laquelle chaque paroi latérale (32) est la plus épaisse dans une zone centrale de la fente dans la direction de l'axe longitudinal.

7. Plaque osseuse (20) selon l'une des revendications 1 à 6, comportant en outre :
un troisième trou (28) formé dans la plaque, le troisième trou étant situé du côté de la première extrémité (34) de la fente ou du côté de la seconde extrémité (36) de la fente.

8. Plaque osseuse (20) selon la revendication 1, la plaque pouvant être pliée au niveau de la première encolure.

9. Plaque osseuse (20) selon la revendication 2, la plaque pouvant être pliée au niveau de la seconde encolure.

10. Plaque osseuse (20) selon l'une des revendications 1 à 2, la plaque pouvant être pliée dans le plan de la plaque et transversalement au plan de la plaque.
